# EUROPEAN PATENT SPECIFICATION

(11) **EP 3 989 866 B1**
(45) Date of publication and mention of the grant of the patent: **22.11.2023**
(21) Application number: 20736477.9
(22) Date of filing: 25.06.2020
(51) Int. Cl.: A61B 90/00, A61N 5/10, A61B 17/00, A61B 34/20

(54) **MARKER FOR MEDICAL IMAGING**
MARKER FÜR MEDIZINISCHE BILDGEBUNG
MARQUEUR POUR IMAGERIE MÉDICALE

(30) Priority: 27.06.2019 NL 2023395
(43) Date of publication of application: 04.05.2022
(73) Proprietor: Nucletron Operations B.V., 3905 TH Veenendaal (NL)
(72) Inventor: SCHUURMAN, Jeroen Anton, 3905 TH Veenendaal (NL); DE JAGER, Wim, 3905 TH Veenendaal (NL); VAN DEN BERG, Martin, 3905 TH Veenendaal (NL); HENNING, Johan, 3905 TH Veenendaal (NL)
(74) Representative: V.O.
(86) International application number: PCT/NL2020/050417
(87) International publication number: WO 2020/263092

(56) References cited:
- WO-A2-01/45578
- WO-A2-2008/009917
- US-A1- 2018 161 121

## Description

### TECHNICAL FIELD

The present disclosure generally relates to brachytherapy and, specifically, to a brachytherapy marker designed for use across multiple imaging modalities.

### BACKGROUND

During high-dose-rate (HDR) brachytherapy treatment, a radioactive source is introduced into or adjacent to a target volume of a patient, for example, a tumor. The radioactive source may be introduced manually or by using an afterloader device. Generally, the afterloader device is used to deliver the radioactive source to a region inside of the patient for a given period of time at pre-determined dwell positions. The radioactive source may be introduced via a cable or catheter and may be positioned within a brachytherapy applicator that is pre-positioned inside of the patient.

Accurate positioning of the radioactive source in the target area of the body is important for administration of the prescribed brachytherapy treatment. For example, if the radiotherapy source is not accurately positioned, then the correct dose of radiation may not be delivered to the intended target area. Additionally, surrounding healthy structures may receive radiation instead of, or in addition to, the intended target region. Exposing the wrong area to radiation may ultimately harm or kill surrounding healthy cells and may leave cancerous cells untreated or undertreated.

To determine whether the radioactive source and/or brachytherapy applicator are positioned correctly and thus to determine whether the radioactive source is introduced to the correct target area-large markers that can be visualized using computed tomography (CT) images are often be used, or an electromagnetic coil for electromagnetic tracking may be used. However, such approaches may not be sufficiently accurate or reliable in determining the correct positioning of the radioactive source. Further, traditional markers may not be useable across multiple different imaging modalities. For example, markers that are compatible with CT imaging may not be compatible for use with magnetic resonance imaging (MRI). Thus, it may be difficult or impossible to use traditional markers during both treatment planning and treatment administration.

WO 2008/009917 A2 discloses a marker comprising an inner ring consisting of titanium and a central opening.

### SUMMARY

The invention is directed to a marker for a medical instrument as defined in claim 1. Further embodiments of the invention are recited in the dependent claims. Embodiments of the disclosure may be drawn to brachytherapy markers. Exemplary markers may include an inner ring consisting of one or more of copper, brass, gold, silver, or titanium; an outer coating consisting of one or more of nickel or iron oxide, wherein a thickness of the outer coating may be about 1 µm to about 30 µm; and a central opening, wherein a diameter of the central opening may be about 0.50 mm to about 3.00 mm.

Various embodiments of the disclosure may include one or more of the following aspects: the inner ring may be copper or brass; the marker may have a toroidal shape; an outer diameter of the marker may be about 1.00 mm to about 5.50 mm; the thickness of the outer coating may be about 4 µm to about 20 µm; or the diameter of the central opening may be about 0.75 mm to about 2.25 mm. The marker may be coupled to a brachytherapy applicator, a brachytherapy needle, a brachytherapy catheter, or a distal region of a brachytherapy transfer tube.

Embodiments of the disclosure may also be drawn to a method of positioning a brachytherapy instrument within a subject. The method may include inserting the brachytherapy instrument within the subject, wherein the brachytherapy instrument includes a marker. The marker may include an inner ring consisting of one or more of copper, brass, gold, silver, or titanium; an outer coating consisting of one or more of nickel or iron oxide, wherein a thickness of the outer coating may be about 1 µm to about 30 µm; and a central opening, wherein a diameter of the central opening may be about 0.50 mm to about 3.00 mm. The method may also include generating a magnetic resonance image of the brachytherapy instrument and the marker when inserted within the subject, wherein the inner ring and the outer coating of the marker may be configured to create an artifact in the image.

Various embodiments of the disclosure may include one or more of the following aspects: adjusting a location of the brachytherapy instrument within the patient based on the artifact created in the image; generating a computed tomography image of the brachytherapy instrument; inserting an afterloader cable within the brachytherapy instrument, wherein the afterloader cable may include an electromagnetic sensor, and detecting the marker using the electromagnetic sensor; inserting a source cable into the brachytherapy instrument and positioning a radioactive source relative to the artefact. In other embodiments, the afterloader cable may be a dummy cable, the source cable may include a second electromagnetic sensor, or the electromagnetic sensor may be a coil.

Embodiments of the present disclosure may also be drawn to a kit comprising a brachytherapy instrument and a marker. The marker may comprise an inner ring consisting of one or more of copper, brass, gold, silver, or titanium; an outer coating consisting of one or more of nickel or iron oxide, wherein a thickness of the outer coating may be about 1 µm to about 30 µm; and a central opening, wherein a diameter of the central opening may be about 0.50 mm to about 3.00 mm; and the marker may have a toroidal shape.

Additional objects and advantages of the disclosed embodiments will be set forth in part in the description that follows, and in part will be apparent from the description, or may be learned by practice of the disclosed embodiments.

It is to be understood that both the foregoing general description and the following detailed description are exemplary and explanatory only and are not restrictive of the disclosed embodiments, as claimed.

### BRIEF DESCRIPTION OF THE DRAWINGS

The accompanying drawings, which are incorporated in and constitute a part of this specification, illustrate various exemplary embodiments and, together with the description, serve to explain the principles of the disclosed embodiments.

For simplicity and clarity of illustration, the figures depict the general structure and/or manner of construction of the various embodiments described herein. For ease of illustration, the figures may depict various components as uniform and smooth shapes. However, a person skilled in the art would recognize that, in reality, the different components may have a non-uniform thickness and/or irregular shapes. Descriptions and details of well-known features (e.g., delivery mechanisms, cables, capsules, catheters, etc.) and techniques may be omitted to avoid obscuring other features. Elements in the figures are not necessarily drawn to scale. The dimensions of some features may be exaggerated relative to other features to improve understanding of the exemplary embodiments. One skilled in the art would appreciate that the cross-sectional views are not drawn to scale and should not be viewed as representing proportional relationships between different regions/layers. Moreover, while certain features are illustrated with straight 90-degree edges or regular planes, in actuality or practice such features may be more "rounded" and gradually sloping.

Further, one skilled in the art would understand that, even if it is not specifically mentioned, aspects described with reference to one embodiment may also be applicable to, and may be used with, other embodiments. There are many embodiments described and illustrated herein, and the present disclosure is neither limited to any single aspect nor embodiment thereof, nor to any combinations and/or permutations of such aspects and/or embodiments. Each aspect of the present disclosure, and/or embodiments thereof, may be employed alone or in combination with one or more of the other aspects of the present disclosure and/or embodiments thereof. For the sake of brevity, certain permutations and combinations are not discussed and/or illustrated separately herein.
FIG. 1 illustrates an exemplary marker according to embodiments of the present disclosure;
FIGS. 2A and 2B illustrate cross-sectional views of exemplary markers according to embodiments of the present disclosure;
FIG. 3 illustrates exemplary markers according to the present disclosure, wherein the markers are placed on an exemplary brachytherapy needle or catheter;
FIG. 4 illustrates an exemplary induction signal report produced by an electromagnetic (EM) sensor device for detecting exemplary markers according to embodiments of the present disclosure;
FIGS. 5A-5D illustrate exemplary 3D images from CT/cone beam computed tomography (CBCT) scans of a catheter of a brachytherapy instrument with markers according to embodiments of the present disclosure;
FIGS. 6A and 6B illustrate exemplary magnetic resonance images of a nitinol marker compared to a nitinol marker having a coating, in accordance with an embodiment of the present disclosure; and
FIG. 7 illustrates a flow chart of an exemplary method of determining an afterloader treatment plan using markers according to the present disclosure.

### DETAILED DESCRIPTION

It should be noted that the description set forth herein is merely illustrative in nature and is not intended to limit the embodiments of the subject matter, or the application and uses of such embodiments. Any implementation described herein as exemplary is not to be construed as preferred or advantageous over other implementations. Rather, the term "exemplary" is used in the sense of example or "illustrative," rather than "ideal." The terms "comprise," "include," "have," "with," and any variations thereof are used synonymously to denote or describe a non-exclusive inclusion. As such, a device or a method that uses such terms does not include only those elements or steps, but may include other elements and steps not expressly listed or inherent to such device and method. Further, the terms "first," "second," and the like, herein do not denote any order, quantity, or importance, but rather are used to distinguish one element from another. The term "distal" refers to the direction that is away from the user or operator and into the subject. By contrast, the term "proximal" refers to the direction that is closer to the user or operator and away from the subject.

The singular forms "a," "an," and "the" include plural reference unless the context dictates otherwise. The terms "approximately" and "about" refer to being nearly the same as a referenced number or value. As used herein, the terms "approximately" and "about" generally should be understood to encompass ± 5% of a specified amount or value. All ranges are understood to include endpoints, e.g., a distance between 1.0 cm and 5.0 cm includes distances of 1.0 cm, 5.0 cm, and all values between.

The term "brachytherapy instrument" may be used to refer to one or more of a radioactive source or a dummy source; a brachytherapy applicator; a cable and/or capsule for moving the radioactive source or the dummy source; or a rod, a transfer tube, a needle, a catheter, an obturator, or a guide wire used during insertion or during use of a radioactive source or a dummy source.

Brachytherapy typically includes both a treatment planning stage and a treatment delivery stage. Using medical three-dimensional (3D) imaging, such as CT, CBCT, and/or MRI, conformal dose plans may be generated for HDR brachytherapy during treatment planning. The conformal dose plan may be executed during the treatment delivery stage, during which a radioactive brachytherapy source may be steered to one or more dwell positions within the body. To do this, an afterloader, e.g., the Flexitron afterloader by Elekta, may be calibrated with the dose planning based on one or more 3D patient images. Afterloader calibration may generally occur by identifying landmarks in medical imaging, such as recognizing needle tips or applicator lumen tips, which may not be well defined or may be difficult to reconstruct in 3D medical images.

By contrast, exemplary embodiments of the present disclosure are drawn to specially designed markers that may be associated with a brachytherapy applicator, needle, or other brachytherapy instrument. These markers may provide for improved measurement of their location in CBCT, CT, and MR images, and may provide for improved measuring by the afterloader, e.g., by the indexer length. A fixed marker position may be defined per applicator or catheter, so that the distance from the beginning of the applicator or catheter (the original zero point for afterloaders like Elekta's Flexitron afterloader) to the marker is a fixed distance according to the type of applicator or catheter used. For example, the indexer length of the afterloader may be zeroed at the marker point in a 3D medical image, which may allow the 3D dwell positions to be scaled to the indexer lengths in the afterloader treatment plans. By integrated exemplary marker(s) into brachytherapy instruments (e.g., applicators, needles, and/or catheters), afterloader source steering may be calibrated to a dose planning system, e.g., the Elekta Oncentra Brachy dose planning system, to an afterloader treatment plan to provide. As a result, embodiments of the present disclosure may provide a more direct and a more precise afterloader calibration.

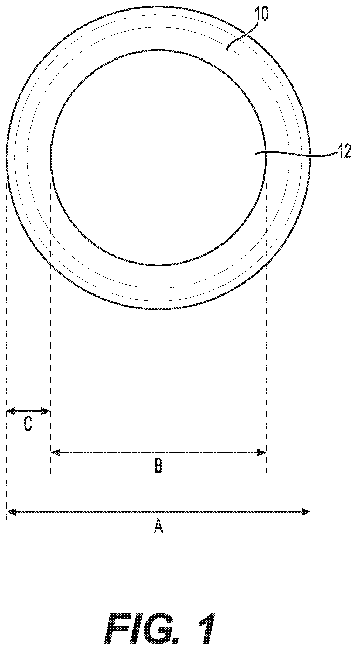

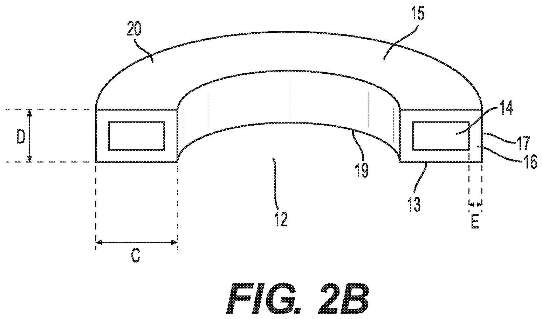

FIG. 1 depicts a marker 10 configured for use with one or more brachytherapy instruments. Marker 10 may be toroidal shaped and may include a central opening 12. One skilled in the art will recognize that marker 10 may have various shapes (as is shown in FIG. 2B). For example, marker 10 may be dimensioned to fit onto or be integrated as part of a brachytherapy catheter or needle, a transfer tube, a catheter connector, and/or a brachytherapy applicator (e.g., along a channel or catheter within the applicator). Accordingly, marker 10 may have an inner diameter (i.e., a diameter of central opening 12) sized to fit around a conventional 2 mm needle or a 3/4 mm catheter of an applicator.

As described above, when performing brachytherapy treatment, it is desirable to achieve accurate placement of the radioactive source. To determine whether an applicator, cable, wire, catheter, needle, or other brachytherapy instrument has been correctly placed within a patient, the instrument may include one or more markers 10 at a known location on the brachytherapy instrument. For example, if a marker 10 is included on a distal region of a wire, catheter, cable, or needle, the distance that the wire, catheter, cable, or needle has been inserted into a patient may be tracked and thus known. In some instances, the location of marker 10 may be imaged (using, e.g., CT imaging) to determine correct placement of a brachytherapy instrument within a subject. An afterloader may then be used to position a radioactive source within the subject so that the source lines up with marker 10. Traditionally, bulky spherical markers may be used so that healthcare personnel can guide the source to a center region of the marker, and software may be used to find the center of the marker for positioning purposes. The use of bulky, spherical markers may not be useful for delivering radiotherapy to smaller regions and/or for use with smaller brachytherapy instruments.

Further, traditional markers may only be compatible with CT images taken during treatment planning and patient preparation, but they may not be compatible with MR imaging, which may be used during treatment administration. As a result, in current brachytherapy workflows, CT scans may be performed after placement of a brachytherapy applicator and before delivery of a radioactive source to determine the correct placement of the applicator, and thus to try to ensure correct placement of the radioactive source during treatment. Positioning of the radioactive source with respect to a target area selected to be treated may be improved if the actual position of the source is monitored directly. This direct monitoring may be made possible by a marker that is designed to be used with both CT imaging during treatment planning and with MRI, e.g., real-time MR imaging, conducted during placement of a brachytherapy instrument and/or during delivery of a radioactive source. Markers according to the present disclosure may address one or more of the problems listed above.

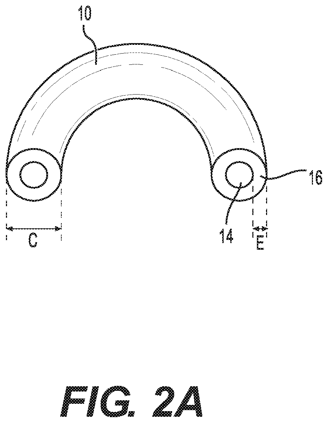

Referring to FIGS. 1 and 2A, an inner diameter of the marker, designated by B, may range from about 0.50 mm to about 3.00 mm, from about 1.00 mm to about 2.50 mm, or from about 1.50 mm to about 2.00 mm. In some embodiments, inner diameter B may range from about 0.75 mm to about 2.25 mm. Depending on what brachytherapy instrument marker 10 is used in conjunction with (e.g., positioned on, within, or otherwise relative to), the inner diameter B may be, for example, about 0.50 mm, about 0.75 mm, about 1.00 mm, about 1.25 mm, about 1.50 mm, about 1.75 mm, about 2.00 mm, about 2.25 mm, about 2.50 mm, about 2.75 mm, or about 3.00 mm.

A thickness of marker 10, designated by C, may range from about 0.25 mm to about 1.25 mm. The thickness C may be, for example, about 0.25 mm, about 0.30 mm, about 0.35 mm, about 0.40 mm, about 0.45 mm, about 0.50 mm, about 0.55 mm, about 0.60 mm, about 0.65 mm, about 0.70 mm, about 0.75 mm, about 0.80 mm, about 0.85 mm, about 0.90 mm, about 0.95 mm, about 1.00 mm, about 1.05 mm, about 1.10 mm, about 1.15 mm, about 1.20 mm, or about 1.25 mm. An outer diameter of marker 10, designated by A, may range from about 1.00 mm to about 5.50 mm, e.g., from about 1.25 mm to about 5.25 mm, from about 1.50 mm to about 5.00 mm, or from about 1.75 mm to about 4.75 mm. In other examples, the outer diameter A may range from about 2.00 mm to about 4.00 mm. The outer diameter may be, for example, about 1.00 mm, about 1.25 mm, about 1.50 mm, about 1.75 mm, about 2.00 mm, about 2.50 mm, about 3.00 mm, about 3.50 mm, about 4.00 mm, about 4.50 mm, about 5.00 mm, about 5.25 mm, or about 5.50 mm.

As discussed above, marker 10 need not be toroidal shaped. For example, one or more surfaces of marker 10 may be flattened and/or may have a change in curvature. In the embodiment of FIG. 2B, a disc-shaped marker 20 has a planar distal surface 13, a planar proximal surface 15, an outer wall 17 extending between the distal surface 13 and the proximal surface 15, and an inner wall 19 extending between the distal surface and the proximal surface. The inner wall 19 may define the central opening of the marker 12. Marker 20 may have similar dimensions as those listed above in regards to marker 10, but marker 20 may have, e.g., a rectangular cross-sectional shape and thus may have a length and a thickness (i.e., width) that are not the same. A length of marker 10, designated by D, may range from about 0.25 mm to about 0.75 mm. The length of the marker may be, for example, about 0.30 mm, about 0.40 mm, about 0.50 mm, about 0.60 mm, or about 0.70 mm. In some embodiments, length D may be different than thickness C (described above), or, in some embodiments, length D and thickness C may have equal dimensions.

Referring to both FIGS. 2A and 2B, markers according to the present disclosure may include an inner ring 14 and an outer coating 16. Inner ring 14 may be copper, brass, gold, silver, titanium, or combinations or alloys thereof. In other examples, inner ring 14 may be copper, brass, or a combination thereof. Outer coating 16 may be nickel, iron, or another ferromagnetic material or combination of ferromagnetic materials such that the outer coating does not produce a large artefact in MRI imaging. In some examples, the outer coating 16 may be, e.g., nickel, iron, cobalt, NiCo, magnetite, or iron oxide. In some embodiments, outer coating 16 may not include magnetic steel. In some examples, outer coating 16 may be comprised solely of nickel.

The thickness of outer coating 16, designated by E, may range from about 1 µm to about 30 µm, e.g., from about 2 µm to about 27 µm, from about 3 µm to about 24 µm, or from about 4 µm to about 20 µm. In some examples, the thickness of outer coating 16 may range from about 2 µm to about 12 µm. The thickness of outer coating 16 may be, for example, about 4 µm, about 6 µm, about 8 µm, about 10 µm, about 12 µm, about 14 µm, about 16 µm, about 18 µm, about 20 µm, about 22 µm, about 24 µm, about 26 µm, about 28 µm, or about 30 µm. The thickness of outer coating 16 may be uniform around inner ring 14.

In some exemplary aspects, an exemplary marker 10 may have an inner ring formed of copper or brass ring-shaped with a diameter of 0.25 mm to 0.5 mm and may have a nickel or iron oxide coating of about 1 µm to about 14 µm, e.g., from about 2 µm to about 12 µm, from about 5 µm to about 10 µm, or from about 6 µm to about 8 µm.

An artefact, when referring to CT and MRI scans, is an anomaly seen during visual representation, wherein the anomaly is not present in the original object. Large artefacts may obscure and/or obstruct MRI scans, such that areas of interest cannot be visually evaluated. A small artefact, however, may be beneficial. For example, a small artefact may provide a landmark relative to the surrounding anatomy so that a radioactive source or dummy source may be accurately directed to a target area. The materials and/or size of marker 10 may be designed to produce a mall artefact in MRI and CT scans and may be compatible with both imaging modalities. For example, copper and brass are good conductors and thus may be useful in CT scans, while nickel, when used in MRI scans, may produce a small, yet visible artefact, compared to other materials, such as magnetic steel, which may produce a large artefact. Accordingly, inner ring 14 may be visible in CT/CBCT scans, while outer coating 16 may be visible in MRI scans. Therefore, a marker 10 according to the present disclosure may be used in both CT/CBCT and MRI scans.

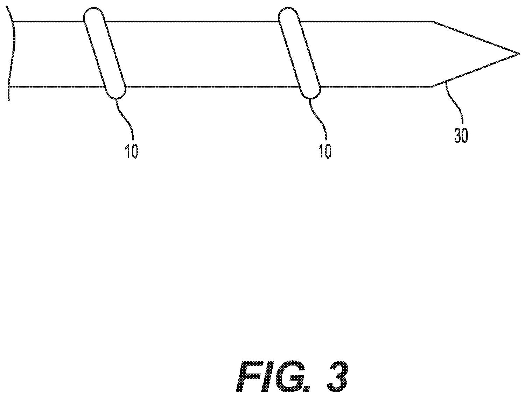

Referring to FIG. 3, one or more markers 10 according to one or more embodiments of the present disclosure may be used with a brachytherapy instrument 30. For example, the central opening of the markers 10 in FIG. 3 may be configured to fit around at least a portion of brachytherapy instrument 30. Brachytherapy instrument 30 may be a needle, catheter, transfer tube, applicator, cable, or guidewire, for example. For example, one or more markers 10 may be associated at a pre-determined location on an applicator, which may be a known distance from a suitable reference point. For example, a marker 10 may be used to indicate a distal end of a relevant structure, such as a cavity in the applicator, or marker 10 may be used to indicate relevant positions, such as source dwell positions, within a brachytherapy instrument. One or more markers 10 may be placed on an applicator to define positions, and the distance from the beginning of the applicator to a marker 10 may be a fixed distance.

In some embodiments, one or more markers 10 may be integrated as part of a breast catheter button for end point detection of a brachytherapy source. In some aspects, one or more markers 10 may be integrated with a transfer tube/catheter connector to help determine that the catheter and/or applicator is correctly connected to the transfer tube. In still other aspects, one or more markers 10 may be integrated at the end of an applicator or catheter for brachytherapy source endpoint detection.

A marker 10 may be included as part of a brachytherapy instrument so that an afterloader may measure the indexer length, which belongs to the position of marker 10. This measurement may be performed via use of electromagnetic induction caused by a conductive ring (e.g., marker 10) and a coil included on (e.g., built into or added onto) one or more of a source cable, check cable, and/or dummy cable. Marker 10 may also be clearly visible in a 3D patient image so that the 3D position of the center of the ring can be measured in a CT, CBCT, and/or MR image by means of a marker point in a dose planning system, e.g., Oncentra Brachy. By this method of measuring the marker using the afterloader source indexer length, and in the patient coordinate system of the 3D image, the afterloader may be calibrated to or with the 3D image of the dose planning. Therefore, it may be possible to more precisely position a brachytherapy source to the pre-determined dwell positions generated by the dose planning.

As described above, one or more markers 10 according to the present disclosure may be used as part of, or in conjunction with, a high dose rate (HDR), a low dose rate (LDR), or a pulsed dose rate (PDR) integrated brachytherapy treatment system. The integrated brachytherapy treatment system may include an afterloader and a radioactive source associated with an electromagnetic coil. Alternatively, instead of an electromagnetic coil, the integrated brachytherapy treatment system may include a Hall sensor associated with the cable and a permanent magnet associated with one or more of the markers 10. In other embodiments, a capacitive sensor may be used in the integrated brachytherapy treatment system to measure changes in capacitance.

In the exemplary embodiment described herein, the afterloader may be configured to measure the induction of the magnetic source caused by the coil. Marker(s) 10 may be integrated as part of one or more brachytherapy instruments, e.g., an applicator, catheter, or needle. Marker(s) 10 may be clearly recognizable on both MRI, CT, and CBCT images. The integrated system may also include a planning system, e.g., an HDR planning system, configured to reconstruct applicators and markers in 3D images, as well as to reconstruct the 3D position(s) of marker(s) 10. The HDR planning system may also be configured to generate dose dwell positions for irradiation. Software associated with the afterloader may be configured to calculate the indexer lengths for the dwell positions of the dose planning. A calibration software module may further be designed to calibrate the indexer length treatment plan to the dose planning using calibration of marker(s) 10 of the afterloader with the planning system.

As shown in FIG. 3 and as discussed above, a marker according to the present disclosure may be coupled to an applicator of a brachytherapy instrument. Once the applicator with the associated marker is inserted into a patient, CT and MRI scans may be performed in real-time because the components of the marker, e.g., the inner ring and the outer coating, may be adapted for use across both imaging modalities, as discussed in detail above. With high-quality 3D imaging of the patient, including CT, CBCT, and/or MRI, more accurate conformal dose plans may be made for HDR brachytherapy.

After creating a conformal dose plan, an HDR brachytherapy source or dummy source may be directed to a pre-determined dwell positions of the treatment planning. To direct the radioactive source, the afterloader may be calibrated with the dose planning based on the patient images. Currently, there is no direct method of calibrating the dose planning to the afterloader treatment planning. In current treatments, this may be done by recognizing needle tips or applicator lumen tips, but the tips may not be well defined or may be difficult to reconstruct in 3D images. In general the accuracy of brachytherapy source positioning may be around +/- 1.5 mm, or with the use of the Flexitron afterloader by Elekta, around +/-0.8 mm. And, for MRI, this type of treatment planning may not be available because of the incompatibility of markers with MR imaging.

Since markers according to the present disclosure may be better defined and visible in 3D images, the positions of the markers may be more accurately measured in the images or by the afterloader, to produce the indexer length. This measurement step may be performed by measuring electromagnetic induction caused by the marker and a coil on a source cable of the afterloader. Since exemplary markers of the disclosure may be visible in the CT and MRI images, the position of the center of the marker may be measured in the image by a marker point in Oncentra^{®} Brachy dose planning. The indexer length of the afterloader may be zeroed at the marker point in the 3D image, by which the 3D dwell positions may be scaled to indexer lengths in the afterloader treatment plan. The marker integrated in the applicator may thus allow the afterloader source to be calibrated to the dose planning system, which may allow for more direct and/or more precise calibration between a dose planning system, like the Oncentra^{®} Brachy dose planning system, and an afterloader treatment plan. The ability to produce CT and MRI images while positioning the applicator may allow the radioactive source to be more accurately directed to the determined dwell positions requested by the dose planning. In some embodiments, this use of direct calibration of the afterloader index length to the 3D patient image may improve the accuracy of brachytherapy source positioning to around approximately +/- 0.5 mm, and MRI calibration may also be available.

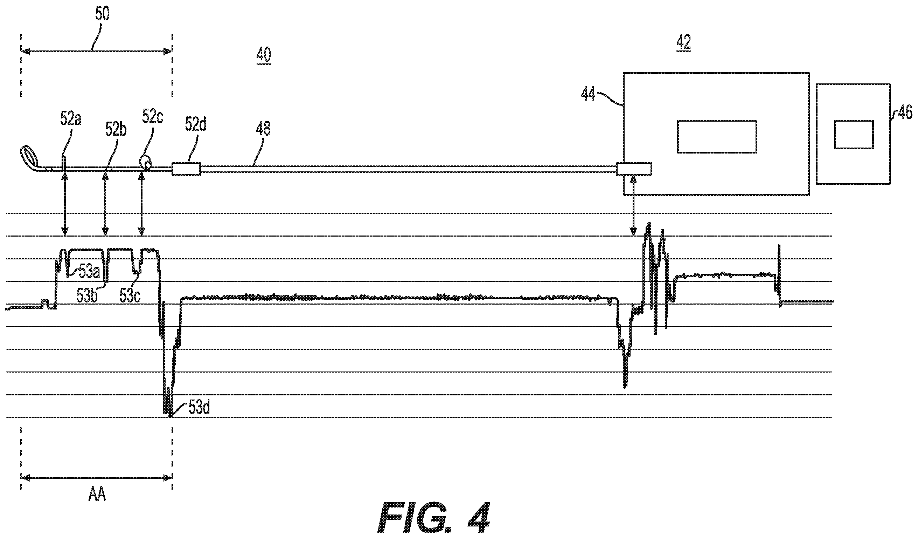

Referring to FIG. 4, the brachytherapy instrument 40 may include an afterloader 42. Afterloader 42 may include a selector 44 and a safe 46. Safe 46 may be located within selector 44. One or more transfer tubes 48 may be used to connect afterloader 42 to an applicator 50, which may be positioned within a subject. An electromagnetic sensor (e.g., a coil) may be located at the end region of a cable. For example, a source cable, a check cable, and/or a dummy cable may include an electromagnetic sensor located at a distal region. In exemplary embodiments in which a source cable includes an electromagnetic sensor, then the sensor may be located, e.g., proximal to or distal to the radioactive source. Either way, the distance between the source and the sensor may be known. The source cable, check cable, and/or dummy cable with an electromagnetic sensor at the distal region may be moved from afterloader 42, through transfer tube 48, and into applicator 50. The sensor (e.g., coil) may be used to measure electromagnetic induction changes as the sensor is moved past the marker(s) of the present disclosure. For example, a copper or brass ring-shaped marker may generate strong changes in electromagnetic induction signals as the cable and accompanying electromagnetic sensor pass though or by the marker, because of the good conductance characteristics of copper and/or brass. Once the electromagnetic source is placed into the applicator, the sensor may detect changes in magnetic induction as the sensor is steered through or by the markers associated with the brachytherapy instrument. The marker(s) may each be located at a dwell position, and thus the electromagnetic signal may indicate respective dwell positions.

For example, the three markers in FIG. 4 may cause corresponding signal changes in the area designated by AA. A marker 52a may cause a change in the measured electromagnetic induction at point 53a, marker 52b may cause a change in the measured electromagnetic induction at point 53b, and marker 52c may cause a change in the measured electromagnetic induction at point 53c. Further, a marker 52d may be located at the distal end of transfer tube 48 causing a change in the measured electromagnetic induction at point 53d to indicate that the cable is being passed from transfer tube 48 and into applicator 50. As discussed above, markers 52a, 52b, 52c may be separate from applicator 50 and may be fitted onto applicator 50 prior to insertion in a patient, or markers 52a, 52b, 52c may be integrated as part of applicator 50, or any other suitable brachytherapy instrument. Markers 52a, 52b, 52c may be oriented adjacent a path to be traveled by an afterloader cable and/or associated radioactive source or may at least partially encircle the path to be traveled by the afterloader cable and/or associated radioactive source. Further, although three markers are depicted in FIG. 4, one of ordinary skill will recognize that fewer (e.g., one or two) or more markers may be associated with applicator 50.

In embodiments in which the electromagnetic sensor is incorporated as part of a dummy cable and/or check cable, then after the locations of the markers have been detected by the sensor, the cable and the electromagnetic sensor device may be withdrawn from applicator 50 and transfer tube 48 and directed back into afterloader 42. The brachytherapy source cable may then be inserted into transfer tube 48 and into applicator 50. In some embodiments, the source cable may also include an electromagnetic sensor (e.g., a coil) proximal to or distal to the radioactive source. In some aspects, a dummy and/or check cable with an electromagnetic sensor may be passed through transfer tube 48 and applicator 50 after the HDR source has been inserted to confirm the targeted location of the HDR source.

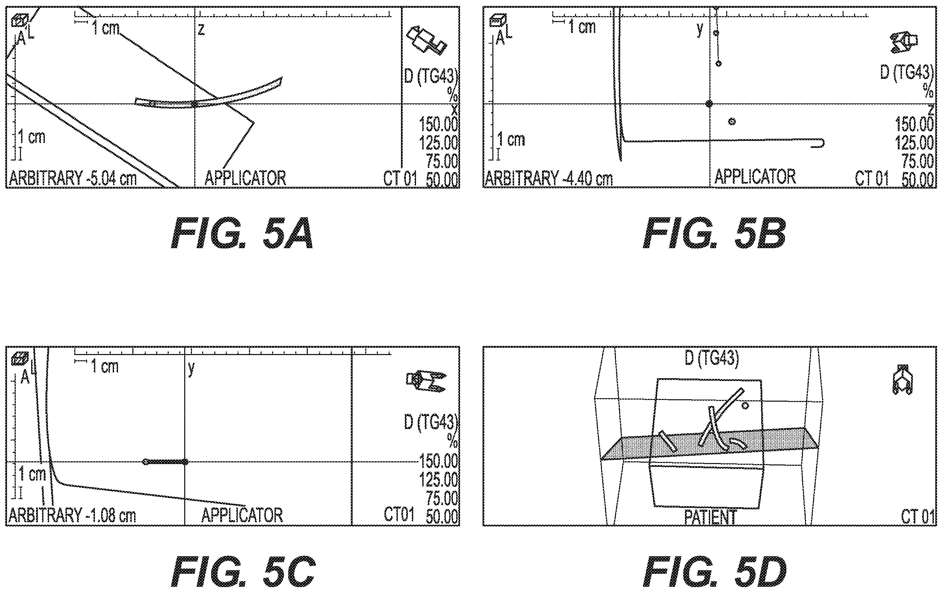

FIGS. 5A-5D illustrate exemplary 3D images from a CT/CBCT scan of a catheter of a brachytherapy device and exemplary markers according to one or more embodiments of the present disclosure. FIGS. 5A-5D depict representations of oblique cross-sectional 3D CT/CBCT images taken with a suitable treatment planning system, such as Oncentra^{®} Brachy by Elekta. Treatment planning systems like Oncentra^{®} Brachy may create a 3D representation of one or more brachytherapy instruments with associated markers by combining multiple two-dimensional (2D) images. For example, a 2D sagittal view (FIG. 5A), a 2D transversal view (FIG. 5B), and a 2D coronal view (FIG. 5C) may be combined to form a 3D image (FIG. 5D). FIG. 5D is a 3D visualization of such catheters. A catheter, e.g., a plastic catheter, and one or more marker rings according to the present disclosure are clearly visible and may be reconstructed as marker points in the dose planning. In CT/CBCT images, the markers (in this instance, copper marker rings) may be visible as white spots (shown in FIGS. 5A-5D as black spots for contrast) with 2800-3000 Hounsfield units (HU). The plastic catheter may be visible with 750 HU, since the plastic may have been improved for CT visibility with BaSO4, and BaSO4 may give a better CT/CBCT contrast when measured in the HU scale. In other CT/CBCT images, the contrast of the markers may be even more distinct, because standard applicator tubes are often made of normal, un-reinforced plastic, so the plastic may appear closer to 100 HU, the air in the catheters may be negative 1000 HU, and the contrast may be 1100 HU.

In an actual CT image, the markers are represented by two white spots, and the catheter may appear as a softer white line. In FIGS. 5A-5C, for clarity, the two markers are depicted as two black spots, and the catheter is represented as an outlined line. In treatment planning software like Oncentra^{®} Brachy, the markers and the optimized dwell positions for dose planning may stored in 3D coordinates and may be used to define the reconstructed catheter. The markers can be used to indicate official marker points as used in the Oncentra^{®} Brachy planning or other suitable treatment planning software.

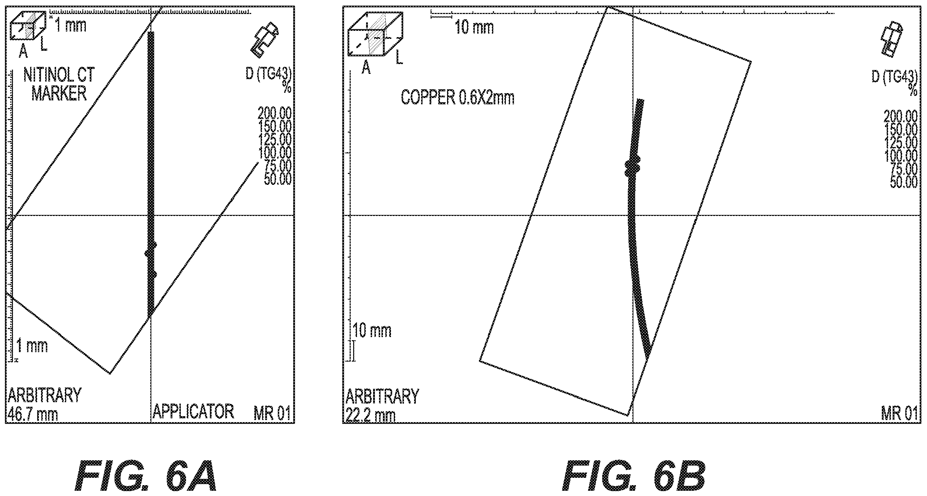

FIGS. 6A-6B compare a standard Nitinol marker (FIG. 6A) to an exemplary marker according to the present disclosure (FIG. 6B). FIG. 6A represents an MR image of a Nitinol marker positioned on a 6F catheter. The Nitinol marker may be located towards a distal end of the catheter at a predetermined location, for example 15 mm, 20 mm, and/or 24 mm from the distal end. These Nitinol markers are generally only used in CT scans. The catheter and Nitinol marker may be inserted into the patient until the marker is at a designated distance within the patient. An afterloader may then be used to place a radioactive source within a catheter so that the source lines up at the center of the marker. As seen in the MR image of FIG. 6A, the Nitinol marker may barely be visible in the dark line which represents the catheter, making it an insufficient marker.

FIG. 6B represents an MR image taken in an oblique plane in which a 6 French (F) catheter is located. The catheter includes a ring-shaped marker formed of copper with a nickel coating, in this case, a 10 µm nickel coating. The marker is clearly visible in the MRI scan of FIG. 6B. The nickel artefact may be a visible, dipole artefact, and the center of the artefact may coincide with the copper ring's center position. Use of MR imaging with the marker of FIG. 6B may allow for a clinician to more accurately steer a brachytherapy source to the target area and to accurately align the source with the central region of the marker. Because the marker may be aligned with a pre-determined dwell position, markers of the present disclosure may increase the accuracy of brachytherapy placement. The accuracy of the marker positioning in Oncentra^{®} Brachy using the marker of FIG. 6B may be equal to or better than +/-0.5 mm.

As discussed above, conventional markers may only be used for CT scans. Markers according to the present disclosure may allow either or both of CT or MRI scans to be performed while simultaneously treating a patient or confirming the position of an applicator or other brachytherapy instrument within the body, e.g., using a dummy or check cable. Such a benefit may allow a healthcare professional administering brachytherapy treatment to determine, e.g., in real time, whether the radioactive source is directed towards the correct location and/or may provide for a more direct measurement of the location of the marker and thus the location of a radioactive or dummy source. In radiation therapy, accurate locations are needed to ensure that normal, healthy tissue is not damaged, and that the targeted, cancerous tissue is treated.

The afterloader software may be integrated with dose planning software, like Oncentra Brachy, to generate the afterloader treatment plan. In such embodiments, the marker position measured in the 3D image by Oncentra Brachy and the result from the electromagnetic induction sensor (e.g., coil) measurement of the afterloader may be taken into account for the calibrated indexer lengths of the dwell positions, as described below.

As discussed above, markers according to the present disclosure may allow for more direct and/or real-time calibration of the afterloader indexer length with the 3D positions of the markers in the patient imaging system. The brachytherapy workflow may be altered to adapt to this new calibration. An extra software module in the afterloader may be configured to handle the calibration, such that the new software module may read the 3D dwell positions from the dose planning system. The 3D position of the marker may be indicated or highlighted, for example, by being shown in a contrasting color, e.g., red, yellow, orange, green, or blue. The indexer length for the marker position may be available from the calibration. The indexer lengths of the dwell positions of the dose planning may be calculated from the distances between the marker point and consecutive dwell positions starting from closest to the marker, or, alternatively, starting from the dwell position farthest from the marker. The calculated indexer lengths may then be stored in the treatment plan of the afterloader.

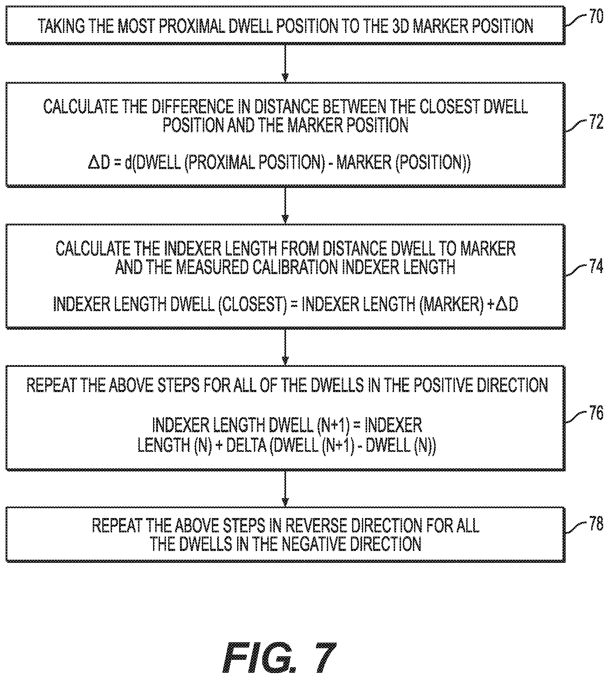

FIG. 7 is a flowchart illustrating a method for determining an afterloader treatment plan according to the present disclosure, using markers according to one or more embodiments of the present disclosure. The method may be performed by an individual processor or by multiple processors in communication with one another. To directly calibrate the source location, a brachytherapy applicator may be placed in a patient. Three-dimensional CT, CBCT, and/or MR images may then be taken of the patient, and the dose planning with dwell positions may be made in the 3D image. The marker positions may be indicated in the 3D image. The patient may be removed from the MRI or CT machine, and the afterloader may then be connected to the brachytherapy applicator. To calibrate the brachytherapy system, a dummy source may be moved to the most distal dwell position (or, in some aspects, the most proximal dwell position). The electromagnetic sensor (e.g., coil), as part of the source, may pass the marker and produce a signal, which may allow the indexer length to be measured by the afterloader.

To perform the steps of FIG. 7, the indexer length of the marker position may be received from the afterloader. Step 70 may include determining the most proximal dwell position relative to the 3D marker position. Step 72 may include calculating the difference in distance between the closest dwell position and the marker position. The distance delta (ΔD) may be equal to ΔD = d (dwell (proximal position) - marker (position)). Step 74 may include calculating the indexer length from distance dwell to the marker and the measured calibration indexer length. In this scenario, indexer length dwell of the closest dwell position = indexer length of the marker + ΔD. Step 76 may include repeating steps 70, 72, and 74, until the indexer lengths of all the dwell positions in the positive direction are calculated. For example, indexer length dwell(n+1) = indexer length(n)+delta(dwell(n+1)-dwell(n)). Step 78 may include repeating steps 70, 72, and 74, to calculate the indexer lengths of all the dwell positions in the negative direction. The same loop may be performed in the reverse direction until all the dwell positions in the negative direction are also determined. It should be recognized by one of skill in the art that dwell positions may be determined in the negative direction and then in the positive direction, as opposed to first in the positive direction and then in the negative direction, and the equations would simply be altered accordingly. The dwell locations may be stored as indexer lengths in the afterloader treatment plan.

In some aspects, rather than obtaining the basis indexer length from the afterloader, the indexer length to the marker may be used as the basis indexer length. In this aspect, in the planning system, the distance from the marker to the dwell position(i) may be measured, which may be the delta(i). The indexer length for dwell position(i) may then be determined. In the scenario, the indexer length(i) may equal the basis indexer length, which may equal the delta(i).

Alternatively, the source may also be calibrated indirectly, e.g., when the marker is integrated into the brachytherapy applicator or needle at a pre-determined distance from the entrance of the brachytherapy instrument (e.g., needle or catheter). For example, a marker may be located 20 cm from a catheter entrance. The source may be steered to 20 cm (with an accuracy of, e.g., 0.25 mm) using a calibration ruler associated with an afterloader, and the indexer length may be stored. This value may be used as the basis indexer length, and from the 3D CT and/or MR image with the deltas(i) per dwell positions(i), the indexer lengths(i) may be calculated. In this way, the indexer lengths may be calculated using the basis indexer length and the ΔD per dwell location as determined from the 3D image.

In some embodiments, at least two markers may be used per catheter or needle, wherein at least one marker is positioned at a distal end of the catheter or needle and at least one marker is positioned at a proximal end of the catheter or needle. The path and stepsize of the afterloader may be controlled and measured. If the marker positions are also visible in the 3D planning image, double direct calibration may be performed. Or, indirect calibration may be performed if both markers are placed in a pre-determined location on the catheter or needle, and the markers are visible in the 3D image.

For any programmable logic, such logic may execute on a commercially available processing platform or a special purpose device. One of ordinary skill in the art may appreciate that embodiments of the disclosed subject matter can be practiced with various computer system configurations, including multi-core multiprocessor systems, minicomputers, mainframe computers, computer linked or clustered with distributed functions, as well as pervasive or miniature computers that may be embedded into virtually any device.

For instance, at least one processor device and a memory may be used to implement the above-described embodiments. A processor device may be a single processor (e.g., associated with an afterloader), a plurality of processors (e.g., associated with an afterloader and/or separate processors used for treatment planning, etc.), or combinations thereof. Processor devices may have one or more processor "cores."

Various embodiments of the present disclosure, as described above in the example of FIG. 7, may be implemented using a processor device. After reading this description, it will become apparent to a person skilled in the relevant art how to implement embodiments of the present disclosure using other computer systems and/or computer architectures. Although operations may be described as a sequential process, some of the operations may in fact be performed in parallel, concurrently, and/or in a distributed environment, and with program code stored locally or remotely for access by single or multiprocessor machines. In addition, in some embodiments the order of operations may be rearranged without departing from the spirit of the disclosed subject matter.

It should be appreciated that the device used for accessing the afterloader treatment platform 80, such as a user device or a source device, may include a central processing unit (CPU). Such a CPU may be any type of processor device including, for example, any type of special purpose or a general-purpose microprocessor device. As will be appreciated by persons skilled in the relevant art, a CPU also may be a single processor in a multi-core/multiprocessor system, such system operating alone, or in a cluster of computing devices operating in a cluster or server farm. A CPU may be connected to a data communication infrastructure, for example, a bus, message queue, network, or multi-core message-passing scheme.

It should further be appreciated that the device used for accessing the afterloader treatment platform 80, such as a user device or a source device, may also include a main memory, for example, random access memory (RAM), and may also include a secondary memory. Secondary memory, e.g., a read-only memory (ROM), may be, for example, a hard disk drive or a removable storage drive. Such a removable storage drive may comprise, for example, a floppy disk drive, a magnetic tape drive, an optical disk drive, a flash memory, or the like. The removable storage drive in this example reads from and/or writes to a removable storage unit in a well-known manner. The removable storage unit may comprise a floppy disk, magnetic tape, optical disk, etc., which is read by and written to by the removable storage drive. As will be appreciated by persons skilled in the relevant art, such a removable storage unit generally includes a computer usable storage medium having stored therein computer software and/or data.

In alternative implementations, secondary memory may include other similar means for allowing computer programs or other instructions to be loaded into a device. Examples of such means may include a program cartridge and cartridge interface (such as that found in video game devices), a removable memory chip (such as an EPROM, or PROM) and associated socket, and other removable storage units and interfaces, which allow software and data to be transferred from a removable storage unit to device.

It should further be appreciated that the device used for accessing the afterloader treatment platform 80, such as a user device or a source device, may also include a communications interface ("COM"). Communications interface allows software and data to be transferred between the afterloader device and external devices. Communications interface may include a modem, a network interface (such as an Ethernet card), a communications port, a PCMCIA slot and card, or the like. Software and data transferred via communications interface may be in the form of signals, which may be electronic, electromagnetic, optical, or other signals capable of being received by communications interface. These signals may be provided to communications interface via a communications path of device, which may be implemented using, for example, wire or cable, fiber optics, a phone line, a cellular phone link, an RF link, or other communications channels.

The hardware elements, operating systems, and programming languages of such equipment are conventional in nature, and it is presumed that those skilled in the art are adequately familiar therewith. A device used for accessing the afterloader treatment platform also may include input and output ports to connect with input and output devices such as keyboards, mice, touchscreens, monitors, displays, etc. Of course, the various server functions may be implemented in a distributed fashion on a number of similar platforms, to distribute the processing load. Alternatively, the servers may be implemented by appropriate programming of one computer hardware platform.

The systems, apparatuses, devices, and methods disclosed herein are described in detail by way of examples and with reference to the figures. The examples discussed herein are examples only and are provided to assist in the explanation of the apparatuses, devices, systems, and methods described herein. None of the features or components shown in the drawings or discussed below should be taken as mandatory for any specific implementation of any of the apparatuses, devices, systems, or methods unless specifically designated as mandatory. In this disclosure, any identification of specific techniques, arrangements, etc. are either related to a specific example presented or are merely a general description of such a technique, arrangement, etc. Identifications of specific details or examples are not intended to be, and should not be, construed as mandatory or limiting unless specifically designated as such. Any failure to specifically describe a combination or sub-combination of components should not be understood as an indication that any combination or sub-combination is not possible. It will be appreciated that modifications to disclosed and described examples, arrangements, configurations, components, elements, apparatuses, devices, systems, methods, etc. can be made and may be desired for a specific application. Also, for any methods described, regardless of whether the method is described in conjunction with a flow diagram, it should be understood that unless otherwise specified or required by context, any explicit or implicit ordering of steps performed in the execution of a method does not imply that those steps must be performed in the order presented but instead may be performed in a different order or in parallel.

Throughout this disclosure, references to components or modules generally refer to items that logically can be grouped together to perform a function or group of related functions. Components and modules can be implemented in software, hardware, or a combination of software and hardware. The term "software" is used expansively to include not only executable code, for example machine-executable or machine-interpretable instructions, but also data structures, data stores and computing instructions stored in any suitable electronic format, including firmware, and embedded software. The terms "information" and "data" are used expansively and includes a wide variety of electronic information, including executable code; content such as text, video data, and audio data, among others; and various codes or flags. The terms "information," "data," and "content" are sometimes used interchangeably when permitted by context.

The above disclosed subject matter is to be considered illustrative, and not restrictive, and the appended claims are intended to cover all such modifications, enhancements, and other implementations, which fall within their scope. Thus, to the maximum extent allowed by law, the scope of the present disclosure is to be determined by the broadest permissible interpretation of the following claims and shall not be restricted or limited by the foregoing detailed description. While various implementations of the disclosure have been described, it will be apparent to those of ordinary skill in the art that many more implementations and implementations are possible within the scope of the disclosure. Accordingly, the disclosure is not to be restricted except in light of the attached claims.

## Claims

1. A marker (10, 20, 52a-52d) for a medical instrument, the marker comprising:
an inner ring (14) consisting of one or more of copper, brass, gold, silver, or titanium; and
a central opening (12), wherein a diameter (B) of the central opening is 0.50 mm to 3.00 mm;
**characterized in that** the marker comprises an outer coating (16) consisting of one or more of nickel or iron oxide, wherein a thickness (E) of the outer coating is 1 µm to 30 µm.

2. The marker of claim 1, wherein the inner ring (14) is copper or brass.

3. The marker of any preceding claim, wherein the marker (10, 20, 52a-52d) has a toroidal
shape.

4. The marker of any preceding claim, wherein an outer diameter (A) of the marker is 1.00 mm to 5.50 mm.

5. The marker of any preceding claim, wherein the thickness (E) of the outer coating is 4 µm to 20 µm.

6. The marker of any preceding claim, wherein the diameter (B) of the central opening is 0.75 mm to 2.25 mm.

7. The marker of any preceding claim, wherein the marker (10) is coupled to a brachytherapy applicator (30).

8. The marker of any preceding claim, wherein the marker (10) is coupled to a brachytherapy needle (30).

9. The marker of any preceding claim, wherein the marker (10) is coupled to a brachytherapy catheter (30).

10. The marker of any preceding claim, wherein the marker (52a-52d) is coupled to a distal
region of a brachytherapy transfer tube (48).

11. The marker of any preceding claim, wherein the inner ring (14) and the outer coating (16) of the marker are configured to create an artifact in the image when a magnetic resonance image of a brachytherapy instrument (50) including the marker (52a, 52b, 52c, 52d) is generated.

12. The marker of claim 11, further comprising means for adjusting a location of the brachytherapy instrument (50) within the patient based on the artifact created in the image.

13. The marker of claims 11 or 12, further comprising:
means for inserting an afterloader cable within the brachytherapy instrument (50), wherein the afterloader cable includes an electromagnetic sensor; and
means for detecting the marker (52a, 52b, 52c, 52d) using the electromagnetic sensor.

14. The marker of claim 13, wherein the afterloader cable is a dummy cable.

15. The marker of claim 14, further comprising means for inserting a source cable into the brachytherapy instrument (50) and positioning a radioactive source relative to the artefact.

16. The marker of claim 15, wherein the source cable includes a second electromagnetic sensor.

17. The marker of claim 13, wherein the electromagnetic sensor is a coil.

## Patentansprüche

1. Marker (10, 20, 52a-52d) für ein Medizinprodukt, wobei der Marker Folgendes umfasst:
einen Innenring (14), der aus Kupfer, Messing, Gold, Silber und/oder Titan besteht; und
eine zentrale Öffnung (12), wobei ein Durchmesser (B) der zentralen Öffnung 0,50 mm bis 3,00 mm beträgt;
**dadurch gekennzeichnet, dass** der Marker eine äußere Beschichtung (16) umfasst, die aus einem oder mehreren von Nickel oder Eisenoxid besteht, wobei eine Dicke (E) der äußeren Beschichtung 1 µm bis 30 µm beträgt.

2. Marker nach Anspruch 1, wobei der Innenring (14) aus Kupfer oder Messing besteht.

3. Marker nach einem der vorstehenden Ansprüche, wobei der Marker (10, 20, 52a-52d) eine Toroid-Form aufweist.

4. Marker nach einem der vorstehenden Ansprüche, wobei ein Außendurchmesser (A) des Markers 1,00 mm bis 5,50 mm beträgt.

5. Marker nach einem der vorstehenden Ansprüche, wobei die Dicke (E) der äußeren Beschichtung 4 µm bis 20 µm beträgt.

6. Marker nach einem der vorstehenden Ansprüche, wobei der Durchmesser (B) der zentralen Öffnung 0,75 mm bis 2,25 mm beträgt.

7. Marker nach einem der vorstehenden Ansprüche, wobei der Marker (10) mit einem Brachytherapie-Applikator (30) gekoppelt ist.

8. Marker nach einem der vorstehenden Ansprüche, wobei der Marker (10) mit einer Brachytherapienadel (30) gekoppelt ist.

9. Marker nach einem der vorstehenden Ansprüche, wobei der Marker (10) mit einem Brachytherapiekatheter (30) gekoppelt ist.

10. Marker nach einem der vorstehenden Ansprüche, wobei der Marker (52a-52d) mit einem distalen Bereich eines Brachytherapie-Übertragungsrohrs (48) gekoppelt ist.

11. Marker nach einem der vorstehenden Ansprüche, wobei der Innenring (14) und die äußere Beschichtung (16) des Markers so konfiguriert sind, dass sie ein Artefakt im Bild erzeugen, wenn ein Magnetresonanzbild eines Brachytherapie-Geräts (50), das den Marker (52a, 52b, 52c, 52d) beinhaltet, erzeugt wird.

12. Marker nach Anspruch 11, ferner umfassend Mittel zum Einstellen einer Position des Brachytherapie-Geräts (50) im Patienten auf der Grundlage des im Bild erzeugten Artefakts.

13. Marker nach Anspruch 11 oder 12, ferner umfassend:
Mittel zum Einführen eines Afterloader-Kabels in das Brachytherapie-Gerät (50), wobei das Afterloader-Kabel einen elektromagnetischen Sensor beinhaltet; und
Mittel zum Erfassen des Markers (52a, 52b, 52c, 52d) unter Verwendung des elektromagnetischen Sensors.

14. Marker nach Anspruch 13, wobei das Afterloader-Kabel ein Blindkabel ist.

15. Marker nach Anspruch 14, ferner umfassend Mittel zum Einführen eines Quellenkabels in das Brachytherapie-Gerät (50) und Positionieren einer radioaktiven Quelle in Abhängigkeit von Artefakt.

16. Marker nach Anspruch 15, wobei das Quellkabel einen zweiten elektromagnetischen Sensor beinhaltet.

17. Marker nach Anspruch 13, wobei der elektromagnetische Sensor eine Spule ist.

## Revendications

1. Marqueur (10, 20, 52a-52d) pour un instrument médical, le marqueur comprenant :
un anneau interne (14) consistant en un ou plusieurs de cuivre, laiton, or, argent, ou titane ; et
une ouverture centrale (12), dans lequel un diamètre (B) de l'ouverture centrale est de 0,50 mm à 3,00 mm ;
**caractérisé en ce que** le marqueur comprend un revêtement externe (16) consistant en un ou plusieurs de nickel ou oxyde de fer, dans lequel une épaisseur (E) du revêtement externe est de 1 µm à 30 µm.

2. Marqueur selon la revendication 1, dans lequel l'anneau interne (14) est du cuivre ou laiton.

3. Marqueur selon l'une quelconque des revendications précédentes, dans lequel le marqueur (10, 20, 52a-52d) présente une forme toroïdale.

4. Marqueur selon l'une quelconque des revendications précédentes, dans lequel un diamètre externe (A) du marqueur est de 1,00 mm à 5,50 mm.

5. Marqueur selon l'une quelconque des revendications précédentes, dans lequel l'épaisseur (E) du revêtement externe est de 4 µm à 20 µm.

6. Marqueur selon l'une quelconque des revendications précédentes, dans lequel le diamètre (B) de l'ouverture centrale est de 0,75 mm à 2,25 mm.

7. Marqueur selon l'une quelconque des revendications précédentes, dans lequel le marqueur (10) est couplé à un applicateur de curiethérapie (30).

8. Marqueur selon l'une quelconque des revendications précédentes, dans lequel le marqueur (10) est couplé à une aiguille de curiethérapie (30).

9. Marqueur selon l'une quelconque des revendications précédentes, dans lequel le marqueur (10) est couplé à un cathéter de curiethérapie (30).

10. Marqueur selon l'une quelconque des revendications précédentes, dans lequel le marqueur (52a-52d) est couplé à une région distale d'un tube de transfert de curiethérapie (48).

11. Marqueur selon l'une quelconque des revendications précédentes, dans lequel l'anneau interne (14) et le revêtement externe (16) du marqueur sont configurés pour créer un artéfact dans l'image lorsqu'une image de résonance magnétique d'un instrument de curiethérapie (50) incluant le marqueur (52a, 52b, 52c, 52d) est générée.

12. Marqueur selon la revendication 11, comprenant de plus un moyen pour ajuster un emplacement de l'instrument de curiethérapie (50) dans le patient sur la base de l'artéfact créé dans l'image.

13. Marqueur selon la revendication 11 ou 12, comprenant de plus :
un moyen pour insérer un câble télécommandé dans l'instrument de curiethérapie (50), dans lequel le câble télécommandé inclut un capteur électromagnétique ; et
un moyen pour détecter le marqueur (52a, 52b, 52c, 52d) en utilisant le capteur électromagnétique.

14. Marqueur selon la revendication 13, dans lequel le câble télécommandé est un câble factice.

15. Marqueur selon la revendication 14, comprenant de plus un moyen pour insérer un câble source dans l'instrument de curiethérapie (50) et positionner une source radioactive par rapport à l'artéfact.

16. Marqueur selon la revendication 15, dans lequel le câble source inclut un second capteur électromagnétique.

17. Marqueur selon la revendication 13, dans lequel le capteur électromagnétique est une bobine.
